# DEMANDE DE BREVET EUROPEEN

(11) **EP 4 527 827 A1**
(43) Date de publication de la demande: **26.03.2025**
(21) Numéro de dépôt: 23199197.7
(22) Date de dépôt: 22.09.2023
(51) Int. Cl.: C07C 45/45, C07D 213/50, C07D 405/06, C07C 45/46

(54) **PROCÉDÉ DE SYNTHÈSE D'UNE BENZOPHÉNONE OU D'UN DÉRIVÉ D'UNE BENZOPHÉNONE**

(71) Demandeur: Centre National de la Recherche, 75016 Paris (FR); Université de Montpellier, 34090 Montpellier (FR); Ecole Nationale Supérieure de Chimie de Montpellier, 34296 Montpellier Cedex 5 (FR)
(72) Inventeur: Taillefer, Marc, 34570 Vailhauques (FR); Wang, Dongjie, 34000 Montpellier (FR)
(74) Mandataire: Ipside

(57) **Abrégé**

L'invention concerne un procédé de synthèse d'une benzophénone ou d'un dérivé d'une benzophénone qui utilise une base métallée avec un solvant choisi parmi les carboxamides linéaires ou cycliques pour activer un composé de formule (II) et permettre son couplage avec un composé de formule (I) de sorte à donner ladite benzophénone ou ledit dérivé de benzophénone.

## Description

### DOMAINE TECHNIQUE DE L'INVENTION

Le domaine de l'invention est celui des voies de synthèse de la benzophénone et de ses dérivés.

Plus précisément, l'invention concerne un procédé de synthèse d'une benzophénone ou d'un dérivé d'une benzophénone

### ÉTAT DE LA TECHNIQUE

La benzophénone est utilisée comme exhausteur et fixateur de parfum ainsi que comme additif pour les plastiques, les revêtements et les formulations adhésives. Elle est également utilisée dans la fabrication d'insecticides, de produits chimiques agricoles, de médicaments hypnotiques, d'antihistaminiques et d'autres produits pharmaceutiques. La benzophénone est aussi utilisée comme agent de fixation des rayons ultraviolets (UV) dans les lunettes de soleil et pour empêcher les UV d'endommager les parfums et les couleurs dans des produits tels que les parfums et les savons. Ajoutée aux emballages en plastique comme bloqueur d'UV, elle permet aux fabricants d'emballer les produits en plastique transparent plutôt que dans des emballages opaques.

Le motif benzophénone est omniprésent en chimie médicinale et on le trouve dans plusieurs molécules d'origine naturelle qui présentent une variété d'activités biologiques, telles que des activités anticancéreuses, anti-inflammatoires, antimicrobiennes et antivirales. En outre, divers motifs synthétiques de benzophénone sont présents dans des médicaments commercialisés. Ils représentent également des ingrédients importants dans les parfums et peuvent agir comme photo-initiateurs.

Différentes voies de synthèse de benzophénones sont aujourd'hui connues :
- la réaction de Friedel-Crafts à partir de chlorure d'aroyle et d'arènes,
- la réaction de couplage croisée, catalysée par des complexes de métaux de transition, entre des halogénures ou pseudohalogénures d'aryles et des dérivés aromatiques pouvant être métallés, en présence de monoxyde de carbone, et
- par couplage oxydant catalysé au palladium entre des dérivés aromatiques porteurs de groupe orienteur et des dérivés aromatiques, en présence de peroxydes.

Les voies de synthèse traditionnelles des benzophénones présentent de nombreux inconvénients : réactifs de départ trop instables, corrosifs, faibles domaines d'application au niveau des benzophénones, emploi des peroxydes dangereux en grande quantité, de catalyseurs de métaux de transition chers et toxiques, emploi de monoxyde de carbone dangereux, etc... Il existe même une méthode industrielle à base de phosgène, un gaz extrêmement toxique à température ambiante (fait partie des gaz de combat).

Il conviendrait donc de trouver une nouvelle méthode d'obtention de la benzophénone et de dérivés de la benzophénone qui s'affranchie des inconvénients précités et avec des risques réduits de toxicité, de pollution et de dangerosité, tout en ayant le plus faible coût possible.

### EXPOSÉ DE L'INVENTION

La présente invention vise à remédier à tout ou partie des inconvénients de l'état de la technique cités ci-dessus.

À cet effet, l'invention vise un procédé de synthèse d'une benzophénone ou d'un dérivé d'une benzophénone comprenant les étapes successives suivantes :
i) Formation d'un milieu réactionnel par addition sous atmosphère dépourvue d'oxygène gazeux, dans un solvant choisi parmi les carboxamides linéaires ou cycliques, d'au moins un composé de formule (I) suivante : 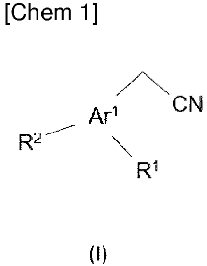 dans laquelle :
   a) Ar¹ est un cycle ou hétérocycle aromatique à cinq ou six chainons, ledit hétérocycle comprenant au moins un atome choisi parmi l'azote N et le phosphore P ;
   b₁) R¹ est identique à R² ou R¹ est un atome d'hydrogène, et R² est choisi parmi :
      - un groupe alkyle linéaire ou ramifié ayant préférentiellement 1 à 6 atomes de carbone, de préférence ayant 1 à 4 atomes de carbone ;
      - un groupe alcényle ou alcynyle linéaire ou ramifié ayant préférentiellement 2 à 6 atomes de carbone, de préférence ayant 2 à 4 atomes de carbone ;
      - un groupe alcoxyle ou thioéther linéaire ou ramifié ayant préférentiellement 1 à 6 atomes de carbone, de préférence ayant 1 à 4 atomes de carbone ;
      - un groupe cycloalkyle avec de préférence 3 à 6 atomes de carbone, préférentiellement un cyclohexyle;
      - un atome d'hydrogène, un hydroxyle, un hydroxyalkyle, un thiol, un carboxyle, un ester, un groupe amido substitué ou non par un ou deux groupes alkyle ou phényle, un formyle, un acyle, un aryle de préférence choisi parmi un phényle et un benzyle, une urée, un isocyanate, un isothiocyanate, un nitrile, un azide, un nitro, une sulfone, un sulfo, un halogène ou un perhalogénoalkyle, un pseudo-halogène ou un trifluorométhyle ;
      ou
   b₂) R¹ et R² sont portés par des atomes adjacents de Ar¹ et forment ensemble, avec lesdits atomes adjacents, un hétérocycle ou un hydrocarbure aromatique polycyclique à deux ou trois cycles, fusionné à Ar¹, ledit hétérocycle ou hydrocarbure aromatique polycyclique à deux ou trois cycles comprenant de préférence un atome d'azote N ;

   ou un de ses sels, et d'au moins un composé de formule (II) suivante : 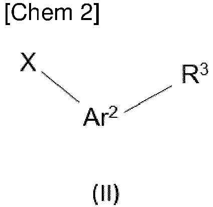
   dans laquelle :
      A) Ar² est un cycle ou hétérocycle aromatique à cinq ou six chainons, ledit hétérocycle comprenant au moins un atome choisi parmi l'azote N, l'oxygène O, le phosphore P et le soufre S, ou Ar² est un hydrocarbure aromatique polycyclique de préférence à deux ou trois cycles, préférentiellement le naphtalène ;
      B) R³ est choisi parmi un groupement alkyle comprenant de préférence 1 à 6 atomes de carbone, un groupement aryle, un groupement aryle éther, un groupement trifluorométhyle, un groupement acétyle, un groupement nitro, un groupement N,N-dialkylsulfonic amide et un halogène ;
      C) X est choisi parmi un atome de Brome, un atome de Chlore, un atome d'Iode et un atome de Fluor ;
   ou un de ses sels ;
ii) Ajout dans le milieu réactionnel d'au moins une base métallée ;
iii) Chauffage du milieu réactionnel à une température T1 comprise entre 0°C et 180°C, de préférence entre 20°C et 160°C, de préférence pendant une durée comprise entre 10 minutes et 24h ;
iv) Mise en contact du milieu réactionnel avec de l'oxygène gazeux à une température T2 comprise entre 0°C et 150 °C de sorte à former ladite benzophénone ou ledit dérivé de la benzophénone, de préférence pendant une durée comprise entre 2 minutes et 2 heures.

Préférentiellement et de manière avantageuse, le procédé objet de la présente invention, par rapport aux méthodes d'obtention de benzophénone et dérivés de benzophénone de l'art antérieur, n'implique pas la mise en oeuvre de catalyseur de métal de transition, de monoxyde de carbone, de peroxyde, de chlorures d'acides et de dérivés aromatiques métallés.

Le procédé objet de la présente invention a un impact amoindri en termes de toxicité, de pollution et de dangerosité par rapport aux méthodes de l'art antérieur, et sa mise en oeuvre représente un coût bien inférieur à celui de ces dernières.

Dans des modes particuliers de mise en oeuvre, l'invention répond en outre aux caractéristiques suivantes, mises en oeuvre séparément ou en chacune de leurs combinaisons techniquement opérantes.

Dans des modes de mise en oeuvre particuliers du procédé objet de la présente invention, l'atmosphère dépourvue d'oxygène gazeux est une atmosphère d'azote ou une atmosphère d'argon.

Dans des modes de mise en oeuvre particuliers du procédé objet de la présente invention, le solvant est choisi parmi les amides monosubstitués et les amides disubstitués.

Dans des modes de mise en oeuvre particuliers du procédé objet de la présente invention, le solvant est choisi parmi le N,N-diméthylacétamide, le N,N-diethylacetamide, le N,N-diméthylformamide et le N-méthyl-2-pyrrolidone.

Dans des modes de mise en oeuvre préférés du procédé objet de la présente invention, R¹ est un atome d'hydrogène et R² est choisi parmi un groupement alkyle linéaire ou ramifié ayant de préférence 1 à 6 atomes de carbone, un groupement alcoxyle dont le groupe alkyle comprend de préférence 1 à 6 atomes de carbone, un groupement aryle de préférence à 6 atomes de carbone et un atome d'halogène, préférentiellement R² est choisi parmi un groupement méthyle, isopropyle, méthoxyle, phényle, un atome de Chlore et un atome de Fluor.

Dans les modes de mise en oeuvre du procédé objet de la présente invention où R¹ est un atome d'hydrogène et R² est un atome d'halogène, l'atome d'halogène est choisi parmi un atome de Chlore, un atome de Fluor, un atome de Brome et un atome d'iode, de préférence parmi un atome de Chlore et un atome de Fluor.

Dans des modes de mise en oeuvre particuliers du procédé objet de la présente invention, lorsque R¹ et R² sont identiques, ils sont choisis parmi un atome d'hydrogène, un groupement méthyle et un atome d'halogène.

Dans des modes de mise en oeuvre particuliers du procédé objet de la présente invention, lorsque R¹ et R² sont identiques et sont un atome d'halogène, ils sont de préférence un atome de Chlore.

Dans des modes de mise en oeuvre particuliers du procédé objet de la présente invention, lorsque R¹ et R² sont portés par des atomes adjacents de Ar¹ et forment ensemble, avec lesdits atomes adjacents, un cycle fusionné à Ar¹, ledit cycle est un dioxolane fusionné à Ar¹.

Dans des modes de mise en oeuvre particuliers du procédé objet de la présente invention, R³ est choisi parmi un groupement méthyl, Phényl, O-Phényl, trifluorométhyle, acétyle, N,N-diisopropylsulfonic amide, un atome de Chlore et un atome de Brome.

Dans des modes de mise en oeuvre particuliers du procédé objet de la présente invention, la base métallée ajoutée en étape ii) est une base comprenant un métal alcalin. La base métallée est de préférence choisie parmi le tertio-butoxyde de potassium, l'amidure de lithium, le méthylate de sodium, l'hydroxyde de potassium et l'hydroxyde de césium.

Dans des modes de mise en oeuvre particuliers du procédé objet de la présente invention, l'étape iii) de chauffage du milieu réactionnel est réalisée à une température T1 de 140°C pendant une durée comprise entre 22h et 24h.

Selon un mode de mise en oeuvre préférée du procédé objet de la présente invention, la température T2 est comprise entre 10°C et 145°C, préférentiellement entre 20°C (appelée également température ambiante) et 140°C. L'étape iv) de mise en contact du milieu réactionnel avec de l'oxygène gazeux peut notamment être mise en oeuvre à une température T2 de 140°C, de préférence pendant 15 minutes. L'étape iv) de mise en contact du milieu réactionnel avec de l'oxygène gazeux peut aussi être mise en oeuvre à une température T2 de 40°C, de préférence pendant 20 minutes. Dans un mode de mise en oeuvre préféré, la température T2 est égale à la température T1.

Dans des modes de mise en oeuvre particuliers du procédé objet de la présente invention, l'étape iv) de mise en contact avec de l'oxygène gazeux est réalisée par mise en contact avec de l'air libre.

Dans des modes de mise en oeuvre particuliers le procédé objet de la présente invention comprend une étape supplémentaire v) de récupération de la benzophénone ou du dérivé de la benzophénone selon une technique classique d'extraction et purification. Par exemple, l'étape v) se fait par extraction avec un solvant organique. Comme exemples plus spécifiques de solvants organiques pouvant être utilisés, on peut notamment citer les hydrocarbures aliphatiques ou aromatiques, halogénés ou non halogénés, les carboxamides et les nitriles. On peut notamment citer le cyclohexane, le toluène, le diméthyl-formamide, l'acétate d'éthyle ou l'acétonitrile.

Dans des modes de mise en oeuvre particuliers du procédé objet de la présente invention, l'étape v) de récupération de la benzophénone ou du dérivé de la benzophénone comprend au moins une sous-étape de précipitation et/ou d'évaporation et/ou de cristallisation.

Dans des modes de mise en oeuvre préférés du procédé objet de la présente invention, l'étape v) de récupération de la benzophénone ou du dérivé de la benzophénone se fait en mettant en oeuvre les sous-étapes suivantes :
- Extraction avec le solvant acétate d'éthyle ;
- Lavage de la phase organique ;
- Séchage ;
- Evaporation du solvant ;
- Purification.
De préférence, le lavage de la phase organique est réalisé avec de la saumure mais il pourrait également être réalisé à l'eau. Le séchage est réalisé de sorte à évacuer l'eau présente dans la phase organique. Selon un mode de mise en oeuvre préféré, le séchage est réalisé sur sulfate de magnésium (MgSO₄) anhydre et est suivi d'une filtration. L'évaporation du solvant est réalisée de préférence sous pression réduite. On obtient en fin d'évaporation un milieu avec les réactifs de départ qui n'ont pas été consommés (soit parce qu'en excès au départ soit parce que la réactivité est insuffisante) ainsi que les produits attendus (et éventuellement des sous-produits). La purification pour obtenir la benzophénone ou du dérivé de la benzophénone purifiée est par exemple réalisée par chromatographie ou distillation.

Dans des modes de mise en oeuvre particuliers du procédé objet de la présente invention, le ratio molaire composé de formule (I) par rapport au composé de formule (II) au sein du milieu réactionnel est compris entre 1/1,2 et 1/2.

Dans des modes de mise en oeuvre particuliers du procédé objet de la présente invention, dans l'étape i) de formation du milieu réactionnel, le composé de formule (I) est ajouté à hauteur de 0,04 % à 0,08 %, de préférence de 0,046 % à 0,077 %, en poids dans le solvant par rapport au poids total du milieu réactionnel, et le composé de formule (II) est ajouté à hauteur de 0,07% à 0,15%, de préférence de 0,075 % à 0,146 % en poids dans le solvant, par rapport au poids total du milieu réactionnel.

Dans des modes de mise en oeuvre particuliers du procédé objet de la présente invention, dans l'étape ii) la base métallée est ajoutée dans le milieu réactionnel à hauteur de 0,08 % à 0,095%, de préférence de 0,085 % à 0,092 %, en poids par rapport au poids total du milieu réactionnel comprenant la base métallée.

### BRÈVE DESCRIPTION DES FIGURES

D'autres avantages, buts et caractéristiques particulières de la présente invention ressortiront de la description non limitative qui suit d'au moins un mode de réalisation particulier des dispositifs et procédés objets de la présente invention, en regard des dessins annexés, dans lesquels :

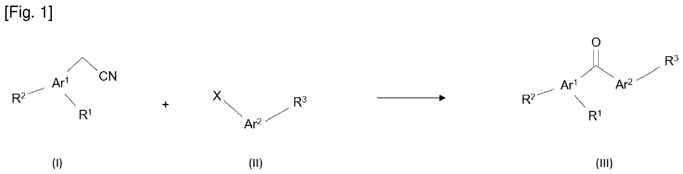

- [Fig. 1] la figure 1 illustre le schéma réactionnel de la réaction mise en oeuvre par le procédé objet de la présente invention faisant réagir le composé de formule (I) avec le composé de formule (II) pour former un composé de formule (III) pouvant être la benzophénone ou un dérivé de la benzophénone.

### DESCRIPTION DÉTAILLÉE DE L'INVENTION

La présente description est donnée à titre d'exemple de réalisation non limitatif.

Comme expliqué ci-dessus, la présente invention vise un procédé de synthèse d'une benzophénone ou d'un dérivé d'une benzophénone. Ce procédé comprend les étapes successives suivantes :
i) Formation d'un milieu réactionnel par addition sous atmosphère dépourvue d'oxygène gazeux, dans un solvant choisi parmi les carboxamides linéaires ou cycliques, d'au moins un composé de formule (I) suivante : 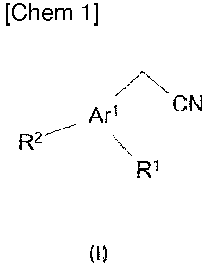 dans laquelle :
   a) Ar¹ est un cycle ou hétérocycle aromatique à cinq ou six chainons, ledit hétérocycle comprenant au moins un atome choisi parmi l'azote N et le phosphore P ;
   b₁) R¹ est identique à R² ou R¹ est un atome d'hydrogène, et R² est choisi parmi :
      - un groupe alkyle linéaire ou ramifié ayant préférentiellement 1 à 6 atomes de carbone, de préférence ayant 1 à 4 atomes de carbone ;
      - un groupe alcényle ou alcynyle linéaire ou ramifié ayant préférentiellement 2 à 6 atomes de carbone, de préférence ayant 2 à 4 atomes de carbone ;
      - un groupe alcoxyle ou thioéther linéaire ou ramifié ayant préférentiellement 1 à 6 atomes de carbone, de préférence ayant 1 à 4 atomes de carbone ;
      - un groupe cycloalkyle avec de préférence 3 à 6 atomes de carbone, préférentiellement un cyclohexyle ;
      - un atome d'hydrogène, un hydroxyle, un hydroxyalkyle, un thiol, un carboxyle, un ester, un group amido substitué ou non par un ou deux groupes alkyle ou phényle, un formyle, un acyle, un aryle de préférence choisi parmi un phényle et un benzyle, une urée, un isocyanate, un isothiocyanate, un nitrile, un azide, un nitro, une sulfone, un sulfo, un halogène ou un perhalogénoalkyle, un pseudo-halogène ou un trifluorométhyle ;
      ou
   b₂) R¹ et R² sont portés par des atomes adjacents de Ar¹ et forment ensemble, avec lesdits atomes adjacents, un hétérocycle ou un hydrocarbure aromatique polycyclique à deux ou trois cycles, fusionné à Ar¹, ledit hétérocycle ou hydrocarbure aromatique polycyclique à deux ou trois cycles comprenant de préférence un atome d'azote N ;

   ou un de ses sels, et d'au moins un composé de formule (II) suivante : 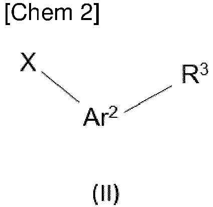
   dans laquelle :
      A) Ar² est un cycle ou hétérocycle aromatique à cinq ou six chainons, ledit hétérocycle comprenant au moins un atome choisi parmi l'azote N, l'oxygène O, le phosphore P et le soufre S, ou Ar² est un hydrocarbure aromatique polycyclique de préférence à deux ou trois cycles, préférentiellement le naphtalene.
      B) R³ est choisi parmi un groupement alkyle comprenant de préférence 1 à 6 atomes de carbone, un groupement aryle, un groupement aryle éther, un groupement trifluorométhyle, un groupement acétyle, un groupement nitro, un groupement N,N-dialkylsulfonic amide et un halogène ;
      C) X est choisi parmi un atome de Brome, un atome de Chlore, un atome d'Iode, et un atome de Fluor ;
   ou un de ses sels ;
ii) Ajout dans le milieu réactionnel d'au moins une base métallée ;
iii) Chauffage du milieu réactionnel à une température T1 comprise entre 0°C et 180°C, de préférence entre 20°C et 160°C, de préférence pendant une durée comprise entre 10 minutes et 24h ;
iv) Mise en contact du milieu réactionnel avec de l'oxygène gazeux à une température T2 comprise entre 0°C 150 °C de sorte à former ladite benzophénone ou ledit dérivé de benzophénone, de préférence pendant une durée comprise entre 2 minutes et 2 heures.

La benzophénone ou le dérivé de la benzophénone ainsi formé, peut par la suite être récupéré selon une technique classique d'extraction et purification.

Le solvant choisi parmi les carboxamides linéaires ou cycliques est de préférence choisi parmi les amides monosubstitués et les amides disubstitués. Il est préférentiellement choisi parmi le N,N-diméthylacétamide, le N,N-diethylacetamide, le N,N-diméthylformamide et le N-méthyl-2-pyrrolidone.

De préférence, lorsque R¹ et R² sont identiques, ils sont choisis parmi un atome d'hydrogène, un groupement méthyle et un atome d'halogène (Fluor, Iode, Chlore, Brome).

Le terme "pseudo-halogène" désigne notamment, les ions cyanures CN, les ions thiocyanate SCN, les ions cyanate OCN-, et les ions azoture N₃⁻.

De préférence, les hydrocarbures aromatiques polycycliques à deux ou trois cycles des formules (I) et (II) sont choisis parmi un naphtalène et un anthracène.

Préférentiellement, lorsque R¹ est un atome d'hydrogène, R² est choisi parmi un groupement alkyle linéaire ou ramifié ayant de préférence 1 à 6 atomes de carbone, un groupement alcoxyle dont le groupe alkyle comprend de préférence 1 à 6 atomes de carbone, un groupement aryle de préférence à 6 atomes de carbone, et un atome d'halogène, de préférence R² est choisi parmi un groupement méthyle, isopropyle, méthoxyle, phényle, un atome de Chlore et un atome de Fluor.

Lorsque R¹ et R² sont portés par des atomes adjacents de Ar¹ et forment ensemble, avec lesdits atomes adjacents, un hétérocycle ou un hydrocarbure aromatique polycyclique à deux ou trois cycles fusionné à Ar¹ et comprenant un atome d'azote N, de préférence ledit hétérocycle ou ledit hydrocarbure aromatique polycyclique à deux ou trois cycles est choisi parmi un cyanométhyl-thiophène, un cyanométhyl-pyroles, un cyanométhyl-pyrazole, un cyanométhyl-phosphole, un 2-(Benzo[b]thiophen-3-yl)acetonitrile, un 2-(3H-Imidazo[4,5-b]pyridin-2-yl)acetonitrile, un 2-(1H-Indol-3-yl)acetonitrile.

Lorsque R¹ et R² sont portés par des atomes adjacents de Ar¹ et forment ensemble, avec lesdits atomes adjacents, un hétérocycle ou un hydrocarbure aromatique polycyclique à deux ou trois cycles fusionné à Ar¹, ledit hétérocycle ou un hydrocarbure aromatique polycyclique à deux ou trois cycles est de préférence un dioxolane fusionné à Ar¹.

R³ est de préférence choisi parmi un groupement méthyl, Phényl, O-Phényl, trifluorométhyle, acétyle, N,N-diisopropylsulfonic amide, un atome de Chlore et un atome de Brome.

De préférence, la formation du milieu réactionnel est telle que le ratio molaire du composé de formule (I) par rapport au composé de formule (II) est compris entre 1/1,2 et 1/2.

Dans l'étape i) de formation du milieu réactionnel, le composé de formule (I) est ajouté à hauteur de 0,04 % à 0,08 %, de préférence de 0,046 % à 0,077 %, en poids dans le solvant par rapport au poids total du milieu réactionnel, et le composé de formule (II) est ajouté à hauteur de 0,07% à 0,15%, de préférence de 0,075 % à 0,146 % en poids dans le solvant, par rapport au poids total du milieu réactionnel.

Différentes bases métallées sont utilisables dans le procédé objet de la présente invention. De préférence, la base métallée est une base comprenant un métal alcalin. Plus préférentiellement, la base métallée est choisie parmi le tertio-butoxyde de potassium, l'amidure de lithium, le méthylate de sodium, l'hydroxyde de potassium et l'hydroxyde de césium.

Dans l'étape ii) la base métallée est ajoutée dans le milieu réactionnel à hauteur de 0,08 % à 0,095%, de préférence de 0,085 % à 0,092 %, en poids par rapport au poids total du milieu réactionnel comprenant la base métallée.

La benzophénone et les dérivés de la benzophénone obtenus par la mise en oeuvre du procédé objet de la présente peuvent être représentés par le composé de formule (III) suivante :

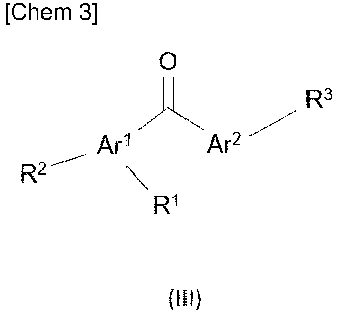

Le schéma de réaction générale selon le procédé objet de la présente invention est illustré en figure 1.

Selon un des mécanismes de réaction possible le composé de formule (II) est déprotoné en α de la fonction nitrile par la base métallée présente dans le milieu réactionnel, pour conduire à l'anion (carbanion) correspondant. Parallèlement, en présence de ce carbanion, du solvant et de la base métallée, le composé de formule (I) est transformé en radical anion lui correspondant qui lui-même évolue en générant un radical aryle. Ce dernier et le carbanion précité se recombinent puis évoluent en présence d'oxygène vers la diarylcétone qu'est la benzophénone ou le dérivé de la benzophénone.

### Exemple de synthèse de la benzophénone selon un mode de mise en œuvre du procédé objet de la présente invention

Pour former le milieu réactionnel, 51,5 mg de benzonitrile (0,5 mmol) en tant que composé de formule (I) et 94,2 mg de bromobenzène (0,6 mmol) en tant que composé de formule (II), sont additionnés sous atmosphère d'argon dans un ballon contenant 1mL de N,N-diméthylacétamide (DMA) utilisé comme solvant. 2,0 mmol de tertio-butoxyde de potassium (KOtBu), en tant que base métallée, sont ensuite additionnés dans le milieu réactionnel dont la température est portée sous agitation à 140°C, pendant 22 heures. Le milieu réactionnel est ensuite mis en contact avec l'air libre pendant 15 minutes supplémentaires à la même température (140°C).
Après extraction avec de l'acétate d'éthyle (trois fois 5 mL), la phase organique est lavée avec de la saumure (trois fois 5 mL), séchée sur sulfate de magnésium (MgSO₄) anhydre et filtrée. Après évaporation du solvant sous pression réduite, le résidu est purifié par chromatographie sur gel de silice (éluant : hexane/dichlorométhane) pour donner la benzophénone avec un rendement isolé de 90% (163,98 mg).

### Exemple de synthèse de dérivé de la benzophénone selon un mode de mise en oeuvre du procédé objet de la présente invention

Le mode opératoire ci-dessus pour la synthèse de la benzophénone est repris avec pour formation de milieu réactionnel, 0,5 mmol d'un dérivé de benzonitrile en tant que composé de formule (I) et 0,6 mmol d'un dérivé de bromobenzène en tant que composé de formule (II), additionnés sous atmosphère d'argon dans un ballon contenant 1mL de N,N-diméthylacétamide (DMA) utilisé comme solvant. 2,0 mmol de tertio-butoxyde de potassium (KOtBu), en tant que base métallée, sont ensuite additionnés dans le milieu réactionnel dont la température est portée sous agitation à 140°C, pendant 22 heures. Le milieu réactionnel est ensuite mis en contact avec l'air libre pendant 15 minutes supplémentaires à la même température (140°C).
Après extraction avec de l'acétate d'éthyle (trois fois 5 mL), la phase organique est lavée avec de la saumure (trois fois 5 mL), séchée sur sulfate de magnésium (MgSO₄) anhydre et filtrée. Après évaporation du solvant sous pression réduite, le résidu est purifié par chromatographie sur gel de silice (éluant : hexane/dichlorométhane) pour donner le dérivé de la benzophénone. Ce procédé donne des dérivés de la benzophénone symétriques (substituants identiques sur les deux noyaux aromatiques) ou dissymétriques (substituants différents sur les deux noyaux aromatiques).

Avec ce même procédé mais en partant d'une bromopyridine à la place du bromobenzène, un dérivé pyridino-benzophénone (benzoylpyridine) est obtenu.

Une liste d'exemples de dérivés (composés de formule (III)) que l'on peut obtenir avec le procédé objet de l'invention en fonction des composés de formules (I) et (II) utilisés, est donnée dans le tableau 1, le tableau 2 et le tableau 3 ci-après :

**[Table 1]**

| **N° de composé de formule (III)** | **Composé de formule (I)** | **Composé de formule (II)** | **Composé de formule (III)** | **Rendement (%)** |
|---|---|---|---|---|
| 1 | 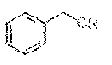 | 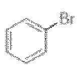 | 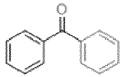 | 90 |
| 2 | 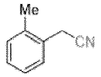 | 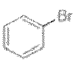 | 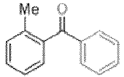 | 51 |
| 3 | 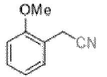 | 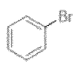 | 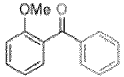 | 33 |
| 4 | 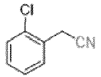 | 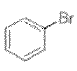 | 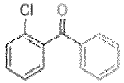 | 50 |
| 5 | 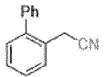 | 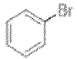 | 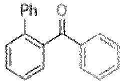 | 77 |
| 6 | 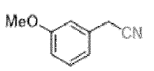 | 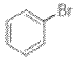 | 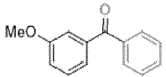 | 51 |
| 7 | 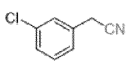 | 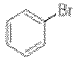 | 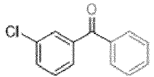 | 36 |
| 8 | 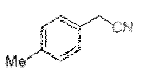 | 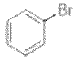 | 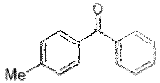 | 61 |
| 9 | 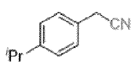 | 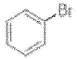 | 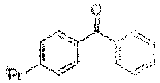 | 54 |
| 10 | 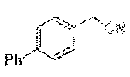 | 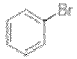 | 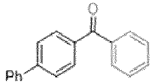 | 66 |
| 11 | 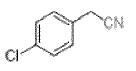 | 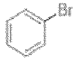 | 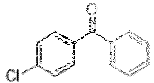 | 41 |
| 12 | 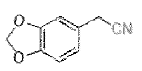 | 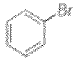 | 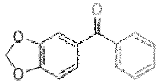 | 55 |
| 13 | 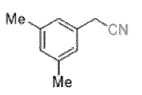 | 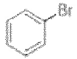 | 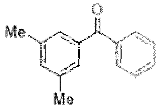 | 48 |
| 14 | 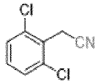 | 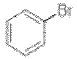 | 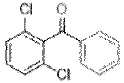 | 33 |
| 15 | 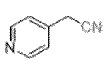 | 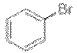 | 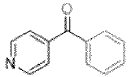 | 39 |
| 15 | 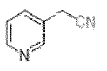 | 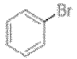 | 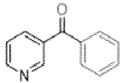 | 47 |
| 17 | 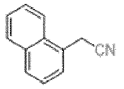 | 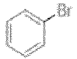 | 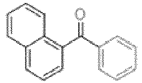 | 42 |

**[Table 2]**

| **N° de composé de formule (III)** | **Composé de formule (I)** | **composé de formule (II)** | **Composé de formule (III)** | **Rendement (%)** |
|---|---|---|---|---|
| 18 | 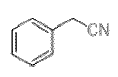 | 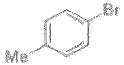 | 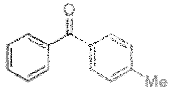 | 47 |
| 19 | 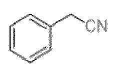 | 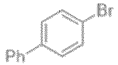 | 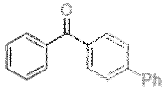 | 50 |
| 20 | 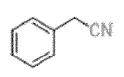 | 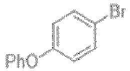 | 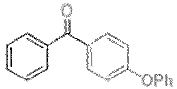 | 55 |
| 21 | 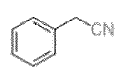 | 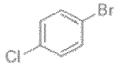 | 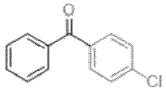 | 63 |
| 22 | 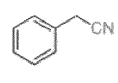 | 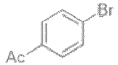 | 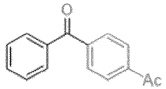 | 41 |
| 23 | 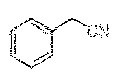 | 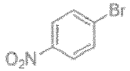 | 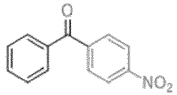 | 45 |
| 24 | 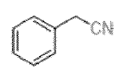 | 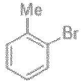 | 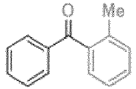 | 62 |
| 25 | 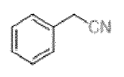 | 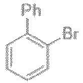 | 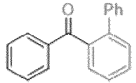 | 41 |
| 26 | 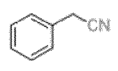 | 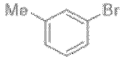 | 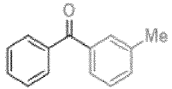 | 80 |
| 27 | 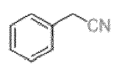 | 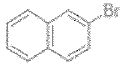 | 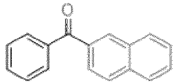 | 79 |
| 28 | 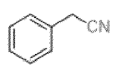 | 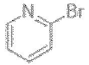 | 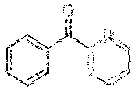 | 64 |
| 29 | 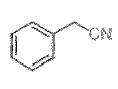 | 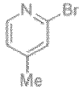 | 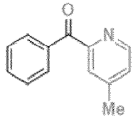 | 57 |
| 30 | 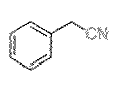 | 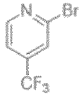 | 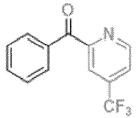 | 55 |
| 31 | 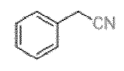 | 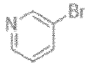 | 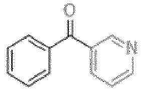 | 72 |

**[Table 3]**

| **N° de composé de formule (III)** | **Composé de formule (I)** | **Composé de formule (II)** | **Composé de formule (III)** | **Rendement (%)** |
|---|---|---|---|---|
| 32 | 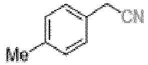 | 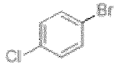 | 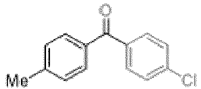 | 67 |
| 33 | 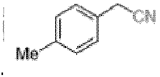 | 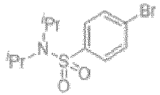 | 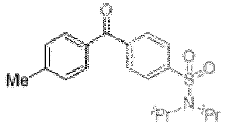 | 67 |
| 34 | 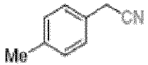 | 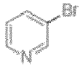 | 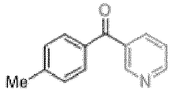 | 76 |
| 35 | 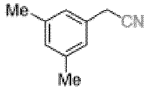 | 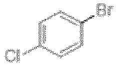 | 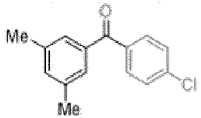 | 69 |
| 36 | 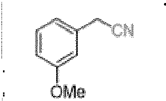 | 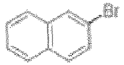 | 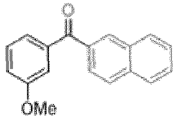 | 44 |
| 37 | 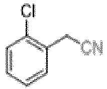 | 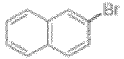 | 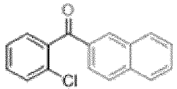 | 55 |
| 38 | 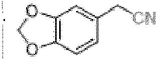 | 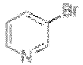 | 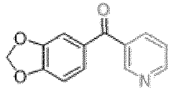 | 51 |
| 39 | 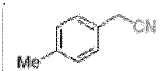 | 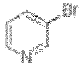 | 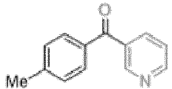 | 76 |

## Revendications

1. Procédé de synthèse d'une benzophénone ou d'un dérivé d'unebenzophénone comprenant les étapes successives suivantes :
i) Formation d'un milieu réactionnel par addition sous atmosphère dépourvue d'oxygène gazeux, dans un solvant choisi parmi les carboxamides linéaires ou cycliques, d'au moins un composé de formule (I) suivante : 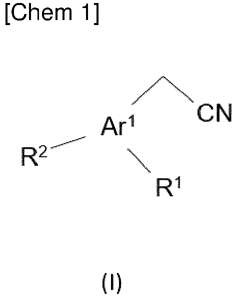 dans laquelle :
a) Ar¹ est un cycle ou hétérocycle aromatique à cinq ou six chainons, ledit hétérocycle comprenant au moins un atome choisi parmi l'azote N et le phosphore P ;
b₁) R¹ est identique à R² ou R¹ est un atome d'hydrogène, et R² est choisi parmi :
- un groupe alkyle linéaire ou ramifié ayant préférentiellement 1 à 6 atomes de carbone, de préférence ayant 1 à 4 atomes de carbone ;
- un groupe alcényle ou alcynyle linéaire ou ramifié ayant préférentiellement 2 à 6 atomes de carbone, de préférence ayant 2 à 4 atomes de carbone ;
- un groupe alcoxyle ou thioéther linéaire ou ramifié ayant préférentiellement 1 à 6 atomes de carbone, de préférence ayant 1 à 4 atomes de carbone ;
- un groupe cycloalkyle avec de préférence 3 à 6 atomes de carbone, préférentiellement un cyclohexyle ;
- un atome d'hydrogène, un hydroxyle, un hydroxyalkyle, un thiol, un carboxyle, un ester, un group amido substitué ou non par un ou deux groupes alkyle ou phényle, un formyle, un acyle, un aryle de préférence choisi parmi un phényle et un benzyle, une urée, un isocyanate, un isothiocyanate, un nitrile, un azide, un nitro, une sulfone, un sulfo, un halogène ou perhalogénoalkyle, un pseudo-halogène ou un trifluorométhyle ;
ou
b₂) R¹ et R² sont portés par des atomes adjacents de Ar¹ et forment ensemble, avec lesdits atomes adjacents, un hétérocycle ou un hydrocarbure aromatique polycyclique à deux ou trois cycles fusionné à Ar¹, ledit cycle comprenant de préférence un atome d'azote N ;
ou un de ses sels, et d'au moins un composé de formule (II) suivante : 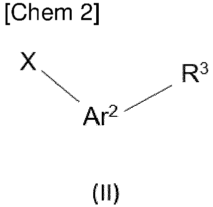
dans laquelle :
A) Ar² est un cycle ou hétérocycle aromatique à cinq ou six chainons, ledit hétérocycle comprenant au moins un atome choisi parmi l'azote N, l'oxygène O, le phosphore P et le soufre S, ou Ar² est un hydrocarbure aromatique polycyclique de préférence à deux ou trois cycles, préférentiellement le naphtalène ;
B) R³ est choisi parmi un groupement alkyle comprenant de préférence 1 à 6 atomes de carbone, un groupement aryle, un groupement aryle éther, un groupement trifluorométhyle, un groupement acétyle, un groupement nitro, un groupement N,N-dialkylsulfonic amide et un halogène ;
C) X est choisi parmi un atome de Brome, un atome de Chlore, un atome d'Iode, un Fluor ;
ou un de ses sels ;
ii) Ajout dans le milieu réactionnel d'au moins une base métallée ;
iii) Chauffage du milieu réactionnel à une température T1 comprise entre 0°C et 180°C, de préférence entre 20°C et 160°C, de préférence pendant une durée comprise entre 10 minutes et 24h ;
iv) Mise en contact du milieu réactionnel avec de l'oxygène gazeux à une température T2 comprise entre 0°C et 150 °C de sorte à former ladite benzophénone ou ledit dérivé de benzophénone, de préférence pendant une durée comprise entre 2 minutes et 2 heures.

2. Procédé selon la revendication 1, dans lequel la base métallée est une base comprenant un métal alcalin.

3. Procédé selon l'une des revendications 1 ou 2, dans lequel la base métallée est choisie parmi le tertio-butoxyde de potassium, l'amidure de lithium, le méthylate de sodium, l'hydroxyde de potassium et l'hydroxyde de césium.

4. Procédé selon l'une quelconque des revendications 1 à 3, dans lequel le solvant est choisi parmi les amides monosubstitués et les amides disubstitués.

5. Procédé selon l'une quelconque des revendications 1 à 4, dans lequel le solvant est choisi parmi le N,N-diméthylacétamide, le N,N-diethylacetamide, le N,N-diméthylformamide et le N-méthyl-2-pyrrolidone.

6. Procédé selon l'une quelconque des revendications 1 à 5, dans lequel R¹ est un atome d'hydrogène et R² est choisi parmi un groupement alkyle linéaire ou ramifié ayant de préférence 1 à 6 atomes de carbone, un groupement alcoxyle dont le groupe alkyle comprend de préférence 1 à 6 atomes de carbone, un groupement aryle de préférence à 6 atomes de carbone, et un atome d'halogène, préférentiellement R² est choisi parmi un groupement méthyle, isopropyle, méthoxyle, phényle, un atome de Chlore et un atome de Fluor.

7. Procédé selon l'une quelconque des revendications 1 à 6, dans lequel R³ est choisi parmi un groupement méthyl, Phényl, O-Phényl, trifluorométhyle, acétyle, N,N-diisopropylsulfonic amide, un atome de Chlore et un atome de Brome.

8. Procédé selon l'une quelconque des revendications 1 à 7, dans lequel le ratio molaire composé de formule (I) par rapport au composé de formule (II) au sein du milieu réactionnel est compris entre 1/1,2 et 1/2.

9. Procédé selon l'une quelconque des revendications 1 à 8, dans lequel dans l'étape i) de formation du milieu réactionnel, le composé de formule (I) est ajouté à hauteur de 0,04 % à 0,08 %, de préférence de 0,046 % à 0,077 %, en poids dans le solvant par rapport au poids total du milieu réactionnel, et le composé de formule (II) est ajouté à hauteur de 0,07% à 0,15%, de préférence de 0,075 % à 0,146 % en poids dans le solvant, par rapport au poids total du milieu réactionnel.

10. Procédé selon l'une quelconque des revendications 1 à 9, comprenant une étape supplémentaire v) de récupération de la benzophénone ou du dérivé de la benzophénone selon une technique d'extraction et purification.
